# EUROPÄISCHE PATENTSCHRIFT

(11) **EP 1 673 074 B1**
(45) Veröffentlichungstag und Bekanntmachung des Hinweises auf die Patenterteilung: **03.01.2007**
(21) Anmeldenummer: 04790428.9
(22) Anmeldetag: 14.10.2004
(51) Int. Cl.: A61K 9/72, A61K 31/7032

(54) **FLÜSSIGE ZUBEREITUNG ENTHALTEND TOBRAMYCIN**
LIQUID PREPARATION CONTAINING TOBRAMYCIN
PREPARATION LIQUIDE CONTENANT DE LA TOBRAMYCINE

(30) Priorität: 15.10.2003 DE 10347995
(43) Veröffentlichungstag der Anmeldung: 28.06.2006
(73) Patentinhaber: PARI GmbH Spezialisten für effektive Inhalation, 82319 Starnberg (DE)
(72) Erfinder: LINTZ, Frank-Christophe, 52222 Stolberg (DE); KELLER, Manfred, 81379 München (DE)
(74) Vertreter: Beckmann, Claus
(86) Internationale Anmeldenummer: PCT/EP2004/011572
(87) Internationale Veröffentlichungsnummer: WO 2005/037256

(56) Entgegenhaltungen:
- WO-A-02/094217
- US-A- 6 083 922
- US-B1- 6 387 886

## Beschreibung

### Technisches Gebiet der Erfindung

Die Erfindung betrifft flüssige Zubereitungen, die das Antibiotikum Tobramycin enthalten und die als pharmazeutische Zubereitungen durch Injektion oder als Aerosol pulmonal oder nasal verabreicht werden können. Sie betrifft ferner pharmazeutische Kits aus zwei Komponenten, aus denen flüssige Zubereitungen zur Verabreichung von Tobramycin hergestellt werden können. Des weiteren betrifft sie die Verwendung der Zubereitungen in pharmazeutischen Produkten, die mit Hilfe eines Verneblers pulmonal oder nasal appliziert und zur Behandlung der Cystischen Fibrose oder anderer Infektionskrankheiten der Atemwege eingesetzt werden können.

### Hintergrund der Erfindung

Tobramycin ist ein Aminoglykosid-Antibiotikum mit der chemischen Bezeichnung O-3-Amino-3-desoxy-α-D-glucopyranosyl(1→4)-O-[2,6-diamino-2,3,6-tridesoxy-α-D-ribo-hexopyranosyl-(1→6)]-2-deoxystreptamin, das sowohl systemisch als auch lokal zur Behandlung schwerer Infektionen eingesetzt wird. Die systemische Behandlung geschieht durch Injektion oder Infusion; angezeigt ist sie bei schweren Infektionen mit einer Reihe von Tobramycin-empfindlichen gramnegativen Bakterien, insbesondere bei Septikämie, Infektionen der unteren Atemwege, des Urogenitalsystems, intraabdominellen Infektionen, Haut-, Weichteil- u. Knocheninfektionen, Osteomyelitis, eitriger Arthritis, bakterieller Endocarditis, gramnegativer Meningitis, sowie bei Infektionen bei immunsupprimierten Patienten.

Bei schweren Infektionen der Atemwege kann Tobramycin inhalativ verabreicht werden. So steht in Deutschland beispielsweise als Fertigarzneimittel TOBI (Fa. Chiron) zur Verfügung, das Tobramycin in Form einer antioxidantienfreien wässrigen Lösung enthält, die als Aerosol inhaliert werden kann. In dieser Form ist der Wirkstoff zur Behandlung von Infektionen der unteren Atemwege mit Pseudomonas aeruginosa bei Patienten mit Mukoviszidose bzw. Cystischer Fibrose geeignet. Weitere Anwendungsgebiete, die sich in der klinischen Prüfung befinden, sind u. a. die Therapie bakterieller Infektionen bei Bronchiektasie und bei Beatmungspatienten sowie bei Tuberkulose.

Mukoviszidose (bzw. Cystische Fibrose) ist eine der häufigsten angeborenen Stoffwechselkrankheiten. Sie ist ein autosomal-rezessiv vererbtes Multiorgansyndrom, hervorgerufen durch einen Mangel an CFTR (cystic fibrosis transmembrane regulator), einem Regulatorprotein des Chlorid-Transports durch die Zellmembran, mit konsekutiver Erhöhung der Viskosität von Körpersekreten. Lokalisiert ist der Enzymdefekt auf Chromosom 7. Der Gendefekt betrifft insbesondere die exokrinen Drüsen mit der Folge, dass in vielen Organen ein zähflüssiger Schleim gebildet wird, der die Lunge, die Bauchspeicheldrüse und die Gallenwege sozusagen verstopft. Etwa 90 Prozent der Probleme betreffen das Atmungsorgan. Eine chronische Lungenentzündung entsteht häufig, wenn der zähe Schleim den Abtransport von Bakterien behindert. Vor allem Pseudomonas aeruginosa tendieren zur Besiedelung der Lunge von Patienten mit Mukoviszidose. Dabei kommt es zu einer Art Teufelskreis: Mit dem Wachstum und der Vermehrung der Bakterien steigt die Sekretion von Schleim und damit die Infektion und Entzündung der Atemwege, und umso schwieriger wird es, die Luftwege mit Sauerstoff zu versorgen. Bei vielen Mukoviszidosepatienten führt die chronische Entzündung der Lunge zu einer schrittweisen Zerstörung des Lungengewebes und zu schweren Atemstörungen, an denen schließlich über 90 % der Patienten sterben.

### Stand der Technik

Für die pulmonalen Behandlung von Mukoviszidosepatienten mit dem Wirkstoff Tobramycin wird heute vor allem das Fertigarzneimittel TOBI eingesetzt. Im Gegensatz zu injektabilen Zubereitungen von Tobramycin enthält TOBI keine stabilisierenden Zusätze von Antioxidantien, die bei der Inhalation Hustenanfälle bzw. Asthma auslösen können.

TOBI wird mit Hilfe eines Vernebler aerosolisiert und eingeatmet. Je nach Bauprinzip und Gerätetyp des Verneblers werden dabei sehr unterschiedliche Ergebnisse erreicht. So hängt die Effizienz der pulmonalen Verabreichung besonders stark von der erzeugten Teilchengröße des Aerosols ab, die je nach Gerät sehr verschieden sein kann. Für die Therapie mit TOBI wird vom Pharmahersteller Chiron der Düsenvernebler PARI LC PLUS^{™} in Kombination mit dem Kompressor Pari Master^{™} (beides Firma PARI) als besonders geeignet empfohlen.

In der Literatur werden weitere Zubereitungen von Tobramycin für die Inhalation beschrieben. US 5,508,269 beschreibt eine Formulierung mit etwa 200 bis 400 mg Tobramycin in einem Volumen von etwa 5 ml. Als isotonisierender Hilfsstoff ist Kochsalz in einem Gehalt von etwa 0,225 % enthalten, und der pH-Wert der Zubereitung ist auf einen Wert von etwa 5,5 bis 6,5 eingestellt. Zur Anwendung soll die Zubereitung mit einem Düsen- oder Ultraschallvernebler zu einem Aerosol mit einer Teilchengröße von 1 bis 5 µm vernebelt werden.

US 6,083,922 beschreibt recht ähnliche Zubereitungen des Wirkstoffs Tobramycin, die allerdings zur Behandlung von Infektionen mit Mycobacterium tuberculosis eingesetzt werden sollen. Etwa 80 bis 300 mg Wirkstoff sollen als Einzeldosis in einem Volumen von etwa 3 bis 5 ml gegeben werden. Der pH-Wert soll auf etwa 5,5 bis 7,0 eingestellt sein, und zur Isotonisierung wird wiederum Kochsalz eingesetzt.

Auch die in US 6,387,886 beschriebene Formulierung von Tobramycin ist ganz ähnlich zusammengesetzt. Sie enthält etwa 250 bis 350 mg Wirkstoff in 5 ml Kochsalzlösung, deren pH-Wert wiederum auf etwa 5,5 bis 6,5 eingestellt ist. Als Verwendung wird die Therapie der chronischen Bronchitis mit Tobramycin-empfindlichen Erregern vorgeschlagen.

WO 03/004005 beschreibt eine Zubereitung mit einem Tobramycin-Gehalt von 75 mg/ml und einem Kochsalzgehalt von 0,45 %. Abweichend von den zuvor zitierten Schriften wird hier allerdings ein pH-Wert zwischen 4,0 und 5,5 gefordert. Als weiteres Merkmal wird ein osmotischer Druck im Bereich von 250 bis 450 mOsmol/l angegeben. In der bevorzugten Ausführungsform hat die Zubereitung einen pH-Wert von 5,2 und einen osmotischen Druck von 280 bis 350 mOsmol/l.

Alle diese Tobramycin-Zubereitungen zeigen in der Praxis diverse Nachteile. Zum einen ist ihre Verträglichkeit nicht sonderlich zufriedenstellend. Dies dürfte vor allem durch den Wirkstoff selbst verursacht und durch den angegriffenen Zustand der Atemwege der Mukoviszidosepatienten verstärkt werden. Zum anderen benötigen Patienten zur Inhalation einer Einzeldosis des Wirkstoffs (am häufigsten ist zur Zeit die zweimal tägliche Inhalation von 300 mg Tobramycin in 5 ml Flüssigkeit) recht lange, nämlich bei den gebräuchlichen Düsenverneblern geräteabhängig etwa 15-20 Minuten. Dies kann insbesondere für schwerkranke Patienten eine deutliche Belastung darstellen. Ein weiterer Nachteil der herkömmlichen Zubereitungen ist ihr von vielen Patienten als schlecht empfundener Geschmack, der natürlich ganz überwiegend durch den Wirkstoff verursacht wird, d.h. durch diejenigen Aerosoltröpfchen, die sich im Mund- und Rachenraum niederschlagen und anschließend - mit Speichel gemischt - die Geschmacksknospen der Zunge erreichen können. Dieses Schicksal betrifft einen erheblichen Anteil aller eingeatmeten Aerosoltröpfchen.

Um zumindest dem Problem der langen Inhalationsdauer und der daraus resultierenden Belastung für die Patienten zu begegnen, schlägt die WO 02/094217 den Einsatz einer höherkonzentrierten Tobramycinlösung vor, durch die eine Einzeldosis aufgrund des geringeren Volumens schneller inhaliert werden kann. Das Applikationsvolumen soll auf maximal 4, besser noch auf nicht mehr als 3,5 ml reduziert werden. Dafür soll die Konzentration des Wirkstoffs auf bis zu etwa 200 mg/ml heraufgesetzt werden, so dass eine Inhalationszeit von weniger als 10 Minuten erreicht wird. Besonders bevorzugt ist eine Wirkstoffkonzentration von 90 bis 120 mg/ml und eine Inhalationszeit von weniger als etwa 6 Minuten. Letztere soll allerdings auch dadurch erreicht werden, dass statt der konventionellen Vernebler modernere Geräte mit besonders hoher Aerosolausbringung eingesetzt werden. Empfohlen werden z. B. stärkere Kompressoren, die an herkömmliche Düsenvernebler angeschlossen werden können, oder piezoelektrische Vernebler, die aufgrund ihres Funktionsprinzips eine höhere Leistung besitzen. Allerdings beschreibt das Dokument in der insgesamt sehr ausführlichen Diskussion der Ausführungsbeispiele lediglich eine einzige Zubereitung, die einen höheren Wirkstoffgehalt als 60 mg/ml aufweist, nämlich eine Zubereitung mit 420 mg Tobramycin in 3,5 ml, entsprechend einer Wirkstoffkonzentration von 120 mg/ml. Diese Zubereitung enthält gleichzeitig einen nicht näher bezeichneten Hilfsstoff zur Einstellung des pH-Wertes auf 6,0 ± 0,5 sowie 0,225 % Kochsalz. Es zeigte sich allerdings, dass diese Zubereitung mit den gewählten Mitteln, die für eine kurze Inhalationszeit optimiert waren, nicht effizient appliziert werden konnte. In einer Klinikstudie war die Menge des im Plasma und im Speichel wiedergefundenen Wirkstoffs nicht größer als nach der Inhalation von 300 mg Tobramycin in Form des Handelspräparats TOBI. So konnte zwar die Inhalationszeit gegenüber TOBI (300 mg) von 18,1 auf 9,7 Minuten verkürzt werden, doch dies nur zu Lasten der Bioverfügbarkeit.

Ein Nachteil der bisher bekannten Zubereitungen von Tobramycin zur Inhalation ist die nicht optimale Verträglichkeit im Respirationstrakt. Gegenüber der Applikation einer vernebelten Placebolösung werden nach der Inhalation von TOBI oder bekannten experimentellen Tobramycin-Zubereitungen gehäuft Reaktion wie Husten und Irritationen der Atemwege beobachtet. Dabei wurde bisher nicht vollständig geklärt, ob es sich um einen reinen Wirkstoffeffekt handelt, der kaum beeinflusst werden kann, oder ob auch die Kombination mit bestimmten, gängigen Hilfsstoffen zu den Unverträglichkeiten beiträgt bzw. einen Beitrag zu ihrer Verringerung leisten kann.

### Beschreibung der Erfindung

Es besteht demnach ein Bedarf an Zubereitungen von Tobramycin für die effiziente, patientengerechte, effektive und verträgliche Inhalation. Insbesondere besteht ein Bedarf an Zubereitungen dieses Wirkstoffs, die rasch und effizient mit leistungsfähigen Inhalatoren verabreicht werden können und dabei dennoch sehr gut verträglich sind, und welche die Nachteile der bekannten Zubereitungen nicht besitzen. Aufgabe der Erfindung ist die Bereitstellung solcher verbesserter Zubereitungen.

Die Aufgabe wird gelöst durch die Bereitstellung von Zubereitungen gemäß Anspruch 1. Weitere Problemlösungen werden aus den übrigen Patentansprüchen und aus der nachfolgenden Beschreibung deutlich. Die Zubereitungen können die pulmonale Antibiotikatherapie von Mukoviscidose-Patienten verbessern; sie lassen sich jedoch auch als Injektionslösungen oder zur lokalen Behandlung von Infektionen im Bereich der oberen Atemwege einsetzen.

Es wird eine sterile, flüssige Zubereitung in Form einer wässrigen Lösung für die Injektion oder Inhalation beansprucht, die pro ml 80 bis 120 mg Tobramycin enthält, ferner einen sauren Hilfsstoff und einen Gehalt an Natriumchlorid von maximal 2 mg/ml aufweist.

Als wässrige Lösung wird hierbei eine Lösung oder kolloidale Lösung verstanden, deren Lösemittel gänzlich oder überwiegend aus Wasser besteht. Steril bedeutet, dass sie hinsichtlich ihrer Sterilität den Anforderungen des jeweils gültigen Europäischen Arzneibuchs (Pharm. Eur.) entspricht. Tobramycin ist die Substanz O-3-Amino-3-desoxy-α-D-glucopyranosyl(1→4)-O-[2,6-diamino-2,3,6-tridesoxy-α-D-ribo-hexopyranosyl-(1→6)]-2-deoxystreptamin einschließlich ihrer Salze, Komplexe, Konjugate und Derivate. Der angegebene Gehalt von etwa 80 bis 120 mg/ml bezieht sich jedoch auf die Base Tobramycin. Dabei ist zu berücksichtigen, dass in der Praxis natürlich leichte Abweichungen vom Nominalgehalt auftreten, die absolut üblich und tolerabel sind. So kann z. B. bei einer Zubereitung mit einem Nominalgehalt von 80 mg/ml ein tatsächlicher Gehalt von 78,5 mg/ml durchaus innerhalb der Produktspezifikation liegen. Dementsprechend ist eine pharmazeutisch tolerable Abweichung bei einem Wirkstoffgehalt im Bereich von 80 bis 120 mg/ml auch im Sinne der Erfindung eingeschlossen.

Der saure Hilfsstoff ist eine physiologisch unbedenkliche Säure oder ein saures Salz, mit dem der pH-Wert der Zubereitung eingestellt wird. Natriumchlorid ist erfindungsgemäß gar nicht oder lediglich in einem Gehalt von maximal 2 mg/ml enthalten, wobei sinngemäß die gleiche Toleranz gilt wie beim Wirkstoffgehalt.

Es wurde gefunden, dass die gemäß Anspruch 1 formulierten Zubereitungen hervorragend geeignet sind, um in gebräuchlichen Verneblern aerosolisiert zu werden. Die Aerosole lassen sich rasch und effizient inhalieren. Insbesondere in Verbindung mit den optionalen Merkmalen, die nachfolgend beschrieben sind, kann eine deutlich verbesserte, patientengerechte Therapie der pulmonalen Infektionen bei Mukoviszidose erreicht werden.

Um das Ziel einer bequemen, sicheren und effizienten Inhalation einer therapeutischen Dosis von Tobramycin zu erreichen, sind verschiedene Einflussparameter zu beachten, von denen einige rezepturtechnischer Art sind. Ein maßgeblicher Parameter ist der Gehalt des Wirkstoffs in der Inhalationslösung. Das Marktprodukt TOBI besitzt mit 300 mg/5 ml einen deutlich niedrigeren Wirkstoffgehalt als den gemäß der vorliegenden Erfindung geforderten. Aufgrund des niedrigen Gehalts ist es kaum möglich, die Inhalationslösung TOBI innerhalb einer kurzen Inhalationszeit zu applizieren. Während eine Inhalationszeit von höchstens etwa 6-8 Minuten wünschenswert erscheint, und eine Inhalationszeit von höchstens etwa 4-5 Minuten ganz besonders wünschenswert ist, um eine hohe Patienten-Compliance zu erzielen, benötigt man für die Inhalation der 5 ml TOBI Lösung in Kombination mit dem laut Gebrauchsinformation empfohlenen Vernebler mindestens etwa 15-20 Minuten. Selbst wenn man von der Geräteempfehlung abweicht und einen leistungsfähigeren Vernebler einsetzt, wird man die angestrebten Inhalationszeiten kaum erreichen, da aufgrund der niedrigen Wirkstoffkonzentration eine relativ große Menge an Flüssigkeit vernebelt werden muss.

Überraschenderweise hat sich nun aber herausgestellt, dass die Wirkstoffkonzentration nicht beliebig hoch innerhalb der Löslichkeit des Wirkstoffs gewählt werden kann, sondern dass ein Wert von etwa 120 mg/ml nicht überschritten werden sollte. So hat sich gezeigt, dass mit einem steigenden Gehalt von Tobramycin zwar die Oberflächenspannung der Lösung durchaus im gewünschten Bereich von etwa 70-76 mN/m gehalten werden kann, dass jedoch die für die Verneblung ebenso wichtige dynamische Viskosität deutlich ansteigt und die Verneblung nachteilig beeinflusst. So besitzen wässrige Tobramycinlösungen mit einem pH von 6,0 bis 6,5 eine etwa 50% höhere Viskosität (ca. 2,9 mPa·s) bei einem Wirkstoffgehalt von. 180 mg/ml gegenüber einer Lösung mit 100 mg/ml (ca. 1,8 mPa·s). Vergleichbare Lösungen mit 120 mg/ml Tobramycin besitzen eine Viskosität von etwa 2,1 mPa·s und können noch fast so effizient vernebelt werden wie Lösungen mit 100 mg/ml. Die Zubereitungen der Erfindung haben unter Normalbedingungen eine Viskosität von etwa 1,4 bis 2,3 mPa·s, und vorzugsweise eine Viskosität im Bereich von etwa 1,6 bis 2,0 mPa·s. Am meisten bevorzugt ist eine Viskosität von etwa 1,8 mPa·s.

Einen besonderen Einfluss auf die lokale Verträglichkeit der Zubereitung hat die Auswahl und Menge des Isotonisierungsmittels. Das im Marktprodukt TOBI enthaltene Kochsalz wird auch in fast allen in der Literatur beschriebenen Zubereitungen eingesetzt oder empfohlen, so z. B. in der WO 03/004005, wo 0,45 % (G/V) Natriumchlorid verwendet werden, und in der WO 02/094217, wo bevorzugt 0,225 % (G/V) zum Einsatz kommen. Aufgrund von Inhalationsversuchen der Erfinder wurde jedoch entdeckt, dass ein möglichst niedriger Kochsalzgehalt zwischen etwa 0,0 und 0,2 % (G/V) verträglicher und mit den übrigen obligatorischen und fakultativen Rezepturbestandteilen bestens kompatibel ist. In einer der bevorzugten Ausführungen der Erfindung ist Kochsalz in einem niedrigeren Gehalt als 0,2 % (G/V) enthalten, insbesondere mit einem Gehalt von 0,17 % (G/V). In einer weiteren Ausführung ist kein Kochsalz enthalten, wobei natürlich die ubiquitären Natriumchloridmengen, die auch in pharmazeutischen Wasserqualitäten enthalten sein können, ausgenommen sind. In einer anderen Ausführung ist als Isotonisierungsmittel ein weitgehend neutrales Salz in der Zubereitung enthalten, bei dem es sich nicht um Kochsalz handelt, sondern z. B. um ein Natriumsulfat oder Natriumphosphat. In diesem Falle sind allerdings andere Salze als Natriumsalze noch mehr zu bevorzugen. So ist von bestimmten Calcium- und Magnesiumsalzen bekannt, dass sie bei der Inhalation von Wirkstofflösungen einen positiven bzw. unterstützenden Einfluss haben können, möglicherweise weil sie selbst den lokalen Irritationen durch die Applikation entgegenwirken und einen bronchodilatatorischen Effekt ausüben, der zur Zeit in der klinischen Literatur postuliert wird (z. B. R. Hughes et al., Lancet. 2003; 361 (9375): 2114-7), und/oder weil sie die Haftung von Keimen an den Proteoglykanen der Schleimhäute des Respirationstrakts hemmen, so dass die mucociliäre Clearance als natürlicher Abwehrmechanismus des Organismus gegen die Erreger indirekt unterstützt wird (K. W. Tsang et al., Eur. Resp. 2003, 21, 932-938). Ganz besonders bevorzugt ist Magnesiumsulfat, das eine ausgezeichnete pulmonale Verträglichkeit besitzt und bedenkenlos inhaliert werden kann, sowie Calciumchlorid (1-10 mMol). Falls der letztgenannte Effekt forciert werden soll, kann auch der Einsatz von Heparin oder Phytohämagglutinin erwogen werden, wobei diese Substanzen natürlich nicht den für die Mineralsalze beschriebenen Beitrag zur Osmolalität bringen können.

Alternativ zu den neutralen Mineralsalzen können als Isotonisierungsmittel auch physiologisch unbedenkliche organische Hilfsstoffe eingesetzt werden. Besonders geeignet sind wasserlösliche Substanzen mit relativ geringer Molmasse, z. B. mit einer Molmasse von unter 300, oder besser noch unter 200, und mit einer entsprechend hohen osmotischen Aktivität. Beispiele für derartige Hilfsstoffe sind Zucker und Zuckeralkohole, insbesondere Mannitol und Sorbitol.

Die Einsatzmenge des gewählten Isotonisierungsmittels ist jeweils so abzustimmen, dass sich unter Berücksichtigung des Gehalts an Tobramycin und des sauren Hilfsstoffs sowie der ggf. weiteren, in der Zubereitung enthaltenen Hilfsstoffe eine Osmolalität von etwa 150 bis 350 mOsmol/l ergibt. Weiterhin bevorzugt ist eine Osmolalität im Bereich von etwa 200 bis 300 mOsmol/l. In einer weiteren Ausführung hat die Zubereitung eine Osmolalität von etwa 230 bis etwa 280 mOsmol/l.

Der saure Hilfsstoff in der Zubereitung dient mehreren Zwecken gleichzeitig. Zum einen wird der pH-Wert auf einen physiologisch gut verträglichen Bereich eingestellt (eine wässrige Lösung von Tobramycin-Base reagiert leicht alkalisch, was für die Inhalation ungünstig ist). Im Sinne der Verträglichkeit sollte die Zubereitung dagegen auf einen pH von etwa 5,0 bis 7,0 eingestellt werden, vorzugsweise auf pH 5,5 bis 6,5. Zum anderen ist ein pH-Wert in den genannten Bereichen von 5,0 bis 7,0 bzw. von 5,5 bis 6,5 besonders vorteilhaft in Hinblick auf die physikochemischen und chemischen Eigenschaften der Zubereitung, insbesondere auf die chemische Stabilität des enthaltenen Wirkstoffs. Sollte ein Kompromiss zwischen einer besonders hohen Stabilität und einer noch akzeptablen Verträglichkeit gewünscht sein, könnte ggf. auch ein pH-Wert im saureren Bereich bis hinunter zu etwa pH 4,0 gewählt werden. Die Verwendung des sauren Hilfsstoffs führt dazu, dass der Wirkstoff Tobramycin in der Zubereitung zumindest teilweise als Salz vorliegt.

Besonders geeignete Hilfsstoffe zur Absenkung des pH-Werts sind starke Mineralsäuren, insbesondere Schwefelsäure und Salzsäure. Des Weiteren kommen auch mittelstarke anorganische oder organische Säuren sowie saure Salze in Frage, z. B. Phosphorsäure, Citronensäure, Weinsäure, Bernsteinsäure, Fumarsäure, Lysin, Methionin, saure Hydrogenphosphate mit Natrium oder Kalium, Milchsäure usw. Am meisten bevorzugt sind jedoch Schwefelsäure und Salzsäure.

Optional kann die Zubereitung eine oberflächenaktive Substanz als Hilfsstoff enthalten. In flüssigen pharmazeutischen Zubereitungen werden Tenside (Surfactants) eingesetzt, um disperse feste oder flüssige Teilchen zu stabilisieren, oder um einen - meist eher schwerlöslichen - Wirkstoff kolloidal zu solubilisieren, z. B. in Form von Micellen, oder als sog. Mikroemulsion. Tenside können sinnvoll sein, um eine bestimmte Oberflächenspannung einzustellen, die für eine optimale und reproduzierbare Verneblung von großer Bedeutung ist.

In einer bevorzugten Ausführung besitzt die erfindungsgemäße Zubereitung unter Normalbedingungen, d. h. bei Raumtemperatur und unter normalem Druck eine Oberflächenspannung von etwa 70 bis 76 mN/m. In einer weiteren Ausführung hat die Zubereitung eine Oberflächenspannung von etwa 72 mN/m. Diese Oberflächenspannungen erlauben eine effiziente Verneblung mit einem hohen Anteil an lungengängigen Tröpfchen mit einem Durchmesser von höchstens 5 µm mit Hilfe gängiger Vernebler. Sie sind auch ohne Zusatz von Tensiden zu erreichen.

Soll die Zubereitung allerdings für den Einsatz in bestimmten Verneblertypen angepasst werden, kann die Oberflächenspannung auf Werte unterhalb von etwa 70 mN/m, ggf. sogar auf Werte unterhalb von etwa 55 mN/m bei Raumtemperatur herabgesetzt werden. Dagegen sollte eine Oberflächenspannung von ca. 30-35 mN/m auch bei Tensidzusatz nicht unterschritten werden. Die durch Tenside herabgesetzte Oberflächenspannung kann dazu beitragen, die Spreitung des Aerosols in der Lunge zu verbessern, was wiederum einen positiven Einfluss auf die Wirksamkeit der Anwendung haben kann.

Der Zusatz von oberflächenaktiven Substanzen kann einen weiteren günstigen Effekt bewirken, wie von den Erfindern überraschend festgestellt wurde: So kann die sensorische Qualität, d. h. insbesondere der Geschmack der Zubereitung bei der Inhalation, durch geeignete Tenside verbessert werden. Als Tenside kommen allerdings nur solche in Frage, die pharmazeutisch einsetzbar und für die pulmonale Applikation geeignet sind. Beispiele für solche Tenside sind Tweens^{®} (insbesondere Tween^{®} 80), Tyloxapol, Vitamin E TPGS und Phospholipide wie z. B. hydrierte Lecithine.

Die Einsatzmengen richten sich nach dem beabsichtigten Effekt: Wenn vor allem eine verbesserte Spreitung in der Lunge beabsichtigt ist, kommen vor allem Tween^{®} 80 und Phospholipide in relativ geringen Konzentrationen von etwa 0,01 bis 0,1 % (GN) in Frage. Soll darüber hinaus der Geschmack des Wirkstoffs maskiert werden, ist die Verwendung von etwas höheren Konzentrationen, z. B. von etwa 0,2 bis 2 % (GN) zu bevorzugen. Besonders bevorzugt ist eine Kombination von Tyloxapol und einem Phospholipid wie Dimyristoylphosphatidylcholin (DMPC), wobei die Konzentration von Tyloxapol bei etwa 0,5 bis 1,5 % (GN), am besten bei etwa 1,0 % (GN), und die von DMPC oder einem vergleichbaren Phospholipid bei etwa 0,2 bis 1,0 % (GN), besonders bevorzugt bei etwa 0,5 % (GN) liegen sollte. Überraschenderweise führt eine solche Kombination von Tensiden keineswegs zu einer erhöhten, sondern eher noch zu einer herabgesetzten Auslösung von lokalen Irritationen im Respirationstrakt bzw. von Bronchokonstriktionen.

Die Kombination von Tyloxapol und einem Phospholipid, insbesondere mit DMPC, ist im Zusammenhang mit dem Wirkstoff Tobramycin auch unter galenischen Gesichtspunkten besonders zu bevorzugen. So ist Tyloxapol allein mit dem Wirkstoff nur begrenzt kompatibel, d. h. es kommt auch innerhalb der wünschenswerten Konzentrationbereiche von Tyloxapol und Tobramycin zu Ausfällungen. Die Inkompatibilitäten können dagegen durch die Kombination mit DMPC verringert bzw. gänzlich vermieden werden.

Auch die Dichte der Zubereitung kann-je nach Art und Konfiguration des Verneblers - einen Einfluss auf die Effizienz der Verneblung haben. Sie sollte zwischen etwa 1,0 und 1,2 g/ml liegen, vorzugsweise zwischen etwa 1,05 und 1,1 g/ml, z. B. bei 1,07 g/ml.

Die Herstellung einer erfindungsgemäßen Zubereitung kann z. B. dadurch geschehen, dass unter aseptischen Bedingungen in einer vorgelegten, abgemessenen Menge von Wasser zu Injektionszwecken nacheinander der saure Hilfsstoff, der Wirkstoff und das Isotonisierungsmittel gelöst werden. Je nach dem, ob und welche Tenside verwendet werden sollen, muss nach deren Zugabe ggf. ein Homogenisierungsschritt erfolgen. In einer der bevorzugten Ausführungen umfasst das Herstellverfahren die Kühlung der Lösung während der bzw. zeitnah zur Auflösung des Wirkstoffs in der wässrigen Phase. Durch diese Maßnahme kann der Wirkstoff zusätzlich stabilisiert und vor Abbau geschützt werden. Aus Stabilitätsgründen kann sich auch ein Arbeiten unter Schutzgasatmosphäre empfehlen.

Die Abfüllung erfolgt vorzugsweise aseptisch in Einzel- oder Mehrdosenbehältnisse. Geeignete Primärverpackungen sind z. B. Polypropylen- oder Polyethylen-Vials (PP-/PE-Vials) und Cycloolefin-Copolymer-Blister (COC-Blister). Versiegelte Kunststoffbehältnisse wie PP- oder PE-Vials lassen sich beipsielsweise vorteilhaft mit dem Blow-Fill-Seal-Verfahren in einem integrierten Prozess formen, befüllen und versiegeln. Die so hergestellten Behältnisse sind insbereonder für flüssige Füllgüter mit einem Volumen ab etwa 0,2 ml geeignet. Besonders patientenfreundlich können sie mit einem Verschluss geformt werden, der durch Drehen oder Abknicken entfernbar ist. Die dabei entstehende Öffnung, durch die der flüssige Inhalt entnehmbar ist, kann so ausgestaltet sein, dass sie auf einen Luer-Anschluss oder Luer-Lock-Anschluss passt. So kann die Öffnung rund gestaltet sein und einen Durchmesser besitzen, der weitgehend dem Außendurchmesser eines männlichen Luer-Anschlusses entspricht. Auf diese Weise könnte eine gewöhnliche Spritze mit Luer-Anschluss mit dem Behältnis schlüssig verbunden werden, beispielsweise um den Inhalt des Behältnisses aufzunehmen und in einen Vernebler zu transferieren, oder um den Inhalt des Behältnisses mit dem Inhalt der Spritze zu mischen und anschließend in einen Vernebler zu geben. Als weitere Alternative kann vorgesehen sein, dass das Kunststoffbehältnis so beschaffen ist, dass es nach Entfernung des Verschlusselements mit einem für die Zufuhr von Flüssigkeit vorgesehenen Anschlussstück eines entsprechend adaptierten Verneblers weitgehend schlüssig verbindbar ist, wodurch eine direkte Einfüllung der Zubereitung in das Reservoir des Inhalators ermöglicht ist.

Kunststoffbehältnisse dieser Art sind weiterhin deshalb vorteilhaft, weil sie leicht mit Prägungen versehen werden können. Hierdurch kann einerseits auf Papieretiketten verzichtet werden, was wünschenswert ist, um die Migration von Bestandteilen des Klebers, des Papiers oder der Druckfarbe durch die Behältniswand in die Zubereitung zu vermeiden. Andererseits können wichtige Information durch eine solche Prägung auch sehbehinderten Patienten zugänglich gemacht werden. Die Prägung kann verschiedene Informationen enthalten, z. B. eine Chargenbezeichnung, ein Haltbarkeitsdatum, eine Produktbezeichnung, Hinweise für die Anwendung, oder auch eine oder mehrere Volumen- oder Dosismarken. Insbesondere für pädiatrische Patienten, bei denen oftmals eine flexible Dosierung nach Alter oder Körpergröße wünschenswert ist, kann eine Mehrzahl von Volumenmarken zur erleichterten Entnahme der gewünschten Dosis ohne weitere Hilfsmittel dienen, wodurch das Risiko von Dosierungsfehlern herabgesetzt werden kann.

In einer weiteren Ausgestaltung der Erfindung werden pharmazeutische Kits bereitgestellt, die jeweils zwei flüssige Komponenten bzw. alternativ eine feste und eine flüssige Komponente in separaten Primärverpackungen innerhalb der gemeinsamen Sekundärverpackung enthalten, wobei die Komponenten so aufeinander abgestimmt sind, dass durch das Kombinieren und Mischen derselben eine gebrauchsfertige erfindungsgemäße Tobramycin-Zubereitung wie voranstehend beschrieben hergestellt werden kann. Dabei enthält die flüssige Komponente bzw. eine der flüssigen Komponenten Lösemittel und ggf. weitere darin enthaltene Hilfsstoffe, während die feste Komponente (bzw. die andere flüssige Komponente) den Wirkstoff (Tobramycin) in konzentrierter und stabilisierter Form enthält. Solche Kits können den Vorteil einer besonders großen pharmazeutischen Stabilität und Lagerfähigkeit besitzen und dennoch im Sinne einer hohen Patientenfreundlichkeit sehr einfach in der Handhabung sein. Alternativ können die Kits so konzipiert sein, dass sie von geschultem medizinischem oder pharmazeutischem Personal (z. B. in der Krankenhausapotheke) in die gebrauchsfertigen Zubereitungen zu überführen sind.

In einer weiteren Variante der Erfindung sind Mehrdosenbehältnisse vorgesehen, die eine Zubereitung wie oben beschrieben enthalten und die des Weiteren so gestaltet sind, dass sie die aseptische Entnahme einer Einzeldosis gestatten. Das Mehrdosenbehältnis kann also wie ein Vial oder eine Infusionsflasche ein Glas- bzw. Kunststoffbehältnis mit einem mit einer Kanüle durchstechbaren Verschluss aus einem Elastomer sein, oder es kann sich um ein komplex aufgebautes Gefäß mit Dosier- und Entnahmevorrichtung handeln.

Einer der besonderen Vorteile von Mehrdosenbehältnissen im Zusammenhang mit Tobramycinzubereitungen für die Inhalation ist die Flexibilität, die problemlos eine individuelle Dosisanpassung gestattet, ohne dass dadurch wesentliche Mengen an Zubereitung verworfen werden müssen, so wie dies bei Einzeldosisbehältnissen nach deren Öffnen der Fall ist. In Krankenhäusern und Pflegeeinrichtungen können auf diese Weise Patienten durch individuelle Dosisanpassung gleichzeitig besonders effizient und potentiell kostengünstig versorgt werden. Ebenso lassen sich spezielle Anforderungen an die Therapie einzelner Patienten besonders leicht berücksichtigen.

Für die Aerosolisierung der Zubereitung kann grundsätzlich jeder in der Therapie einsetzbare Vernebler verwendet werden. Die altbewährten Düsenvernebler sind prinzipiell ebenso geeignet wie neuere Ultraschall- oder piezoelektrische Vernebler, allerdings nachteiliger hinsichtlich der Inhalationszeit. Der Vorteil der Düsenvernebler ist, dass sie bereits einen sehr großen Verbreitungsgrad besitzen und relativ kostengünstig zu beschaffen sind. Viele Patienten sind bereits im Umgang mit gängigen Düsenvernebler versiert. Einige Düsenvernebler der jüngeren Generation (z. B. PARI LC PLUS^{®} und PARI LC STAR^{®}) verwenden Mechanismen, mit denen die Verneblung an den Atmungsrhythmus des Patienten angepasst wird, so dass ein möglichst großer Anteil des erzeugten Aerosols auch für die Inhalation verfügbar ist.

Besonders bevorzugt jedoch ist die Aerosolisierung der Zubereitung mit Hilfe eines modernen piezoelektrischen Verneblers, insbesondere mit dem Vernebler vom Typ eFlow^{™} der Firma PARI. Der besondere Vorteil für den Patienten bei der Verwendung dieses Geräts (oder eines ähnlichen Geräts) ist die gegenüber alternativen Methoden erheblich verkürzte Inhalationszeit. Das Gerät aerosolisiert nicht nur eine größere Menge an Flüssigkeit pro Zeiteinheit, es erzeugt auch ein qualitativ besonders hochwertiges Aerosol mit hohem Anteil an kleinen, lungengängigen Aerosoltröpfchen.

Entscheidend für den therapeutischen Erfolg ist die zuverlässige und adäquate Verfügbarkeit des Wirkstoffs in der Lunge. Patientengerecht ist es, dies innerhalb eines akzeptablen Zeitraums zu bewirken. Von Patienten werden möglichst kurze Inhalationszeiten bevorzugt, und Inhalationszeiten von mehr als etwa 6-8 Minuten könnten die Compliance bereits negativ beeinflussen. Besonders wenig wünschenswert sind Inhalationszeiten von mehr als etwa 10 Minuten. Dagegen sind Inhalationszeiten von weniger als etwa 5-6 Minuten aus der Sicht der Patienten ausgesprochen wünschenswert.

In der herkömmlichen Therapie mit Tobramycin wird das Präparat TOBI, das 300 mg Tobramycin in 5 ml wässriger Lösung enthält, mit einem in der Gebrauchsinformation empfohlenen Düsenvernebler, dem PARI LC PLUS^{®} vernebelt, wofür in der Praxis ein recht langer Zeitbedarf von etwa 15-20 Minuten notwendig ist. Dabei beträgt die lungengängige Fraktion von Aerosoltröpfchen mit weniger als 5 µm Durchmesser etwa 60 % des erzeugten Aerosols (gemessen durch Laser Diffraction mit einem MasterSizer X, Fa. Malvern). Unter Abzug aller Wirkstoffverluste, die im Vernebler, durch die Ausatmung des Patienten und durch die Deposition des Aerosols im oberen Bereich des Respirationstraktes geschehen, kann man davon ausgehen, dass nur etwa 60-80 mg Tobramycin die Lunge des Patienten erreichen (Respirable Dose, RD).

Dagegen werden bei einer erfindungsgemäßen Zubereitung in Kombination mit einem piezoelektrischen Vernebler deutlich höhere Ausstoßraten erzeugt. Dies trifft Insbesondere auf die Verneblung mit dem Schwingmembranvernebler vom Typ eFlow^{™} zu, der gleichzeitig auch höhere Anteile an lungengängigen Aerosoltröpfchen erzeugt, nämlich etwa 75 %. Außerdem sind die konstruktionsbedingten Verluste innerhalb des Gerätes geringer als beim Düsenvernebler. Infolge dessen reicht die Verneblung einer geringeren Wirkstoffmenge aus, um die gleiche Wirkstoffdosis in der Lunge verfügbar zu machen. So kann z. B. aufgrund von In-vitro-Daten davon ausgegangen werden, dass eine erfindungsgemäße Zubereitung mit nur 200 mg Tobramycin in 2 ml Inhalationslösung nach Verneblung mit dem eFlow^{™}-Gerät zu einer Verfügbarkeit von etwa 70-80 mg Wirkstoff in der Lunge (Respirable Dose, RD) führt, also bioäquivalent zur herkömmlichen Therapie mit 5 ml TOBI 300 mg sein dürfte. Der besondere Patientenvorteil liegt nun vor allem in der kurzen Zeit, die für die Aerosolisierung und Inhalation der 2 ml der erfindungsgemäßen Zubereitung mit dem eFlow^{™}-Gerät notwendig ist: In einer In-vitro-Versuchsanordnung geschieht dies innerhalb von etwa 3-4 Minuten, in der Praxis werden hierfür etwa 4-5 Minuten benötigt, in jedem Fall aber weniger als 6 Minuten, was einen erheblichen Unterschied zur herkömmlichen Therapie darstellt.

Es ist deshalb erfindungsgemäß bevorzugt, die Zubereitung hinsichtlich ihrer pharmazeutischen und insbesondere physikochemischen Parameter möglichst optimal auf die Verneblung mit einem piezoelektrischen Vernebler bzw. Schwingmembranvernebler wie dem eFlow^{™} zu formulieren, so dass die Patienten einen besonders hohen Nutzen durch eine möglichst stark verkürzte Inhalationszeit erfahren.

In einer weiteren Ausführung wird die Zubereitung auf die Applikation als Aerosol zur Behandlung der oberen Atemwege abgestimmt. Auch hier besteht die Möglichkeit, Infektionen mit Tobramycin-empfindlichen Erregern lokal zu therapieren. Insbesondere die Schleimhaut der Nasenhöhlen, der Mundhöhle, aber auch der Neben-, Kiefer- und Stirnhöhlen sind grundsätzlich der Aerosoltherapie zugänglich. Die Mund- und Nasenschleimheut kann dabei am leichtesten vom Aerosol erreicht werden. Bereits mechanische Zerstäuber, wie sie häufig für Nasen- oder Mundsprays eingesetzt werden, können in diesem Fall eingesetzt werden. Speziell adaptierte Düsen-, Ultraschall- oder piezoelektrische Vernebler können dagegen für eine wesentlich bessere Benetzung der Mund- oder Nasenschleimhaut mit der aerosolisierten Zubereitung sorgen.

Schwieriger ist die effiziente Applikation eines Aerosols in die weniger ventilierten Hohlräume des oberen Respirationstraktes. Andererseits sind gerade die Stirn- und Nasennebenhöhlen sehr häufig Ort einer Infektion. Gewöhnlich wird versucht, solche Infektionen mit Expektorantien und schleimhautabschwellenden Mitteln zu behandeln, was nicht immer erfolgreich ist. Die schwereren Fälle werden zusätzlich durch eine systemische Antibiotikatherapie behandelt, die jedoch nicht von allen Patienten gut vertragen wird.

Die einfache nasale Inhalation einer aerosolisierten Wirkstoffzubereitung führt diese zwar in die Nähe der Nebenhöhlen, jedoch passiert der Aerosolstrom ganz überwiegend die Öffnungen (Ostien) zu den Nebenhöhlen, ohne dass ein wesentlicher Anteil des Aerosols in die Nebenhöhlen eintritt.

Seit kurzem stehen jedoch speziell adaptierte Düsenvernebler zur Verfügung, mit denen die Nebenhöhlen wesentlich besser als bisher erreicht werden können. Diese Vernebler besitzen einerseits ein Nasenstück zur Lenkung des Aerosolstroms in die Nase. Falls nur ein Nasenloch zur Inhalation des Aerosols verwendet wird, muss das andere durch eine geeignete Vorrichtung verschlossen werden. Ferner sind diese Vernebler dadurch gekennzeichnet, dass sie ein Aerosol mit pulsierendem Druck ausstömen lassen. Die pulsierenden Druckwellen sorgen für eine intensivierte Ventilation der Nebenhöhlen, so dass ein gleichzeitig inhaliertes Aerosol sich besser in diese Hohlräume verteilen kann. Beispiele für entsprechende Verneblervorrichtungen werden in DE 102 39 321 B3 offenbart. In einer der bevorzugten Ausführungen wird die Zubereitung der Erfindung zur Herstellung eines Arzneimittels zur Applikation mit Hilfe einer der dort beschriebenen Vorrichtungen zur Behandlung von Infektionen des oberen Respirationstrakts verwendet, insbesondere mit einem Gerät vom Typ PARI Sinus.

### Beispiele

Die nachfolgenden Ausführungsbeispiele dienen der Veranschaulichung der Erfindung in einigen ausgewählten Ausgestaltungsmöglichkeiten.

### Beispiel 1: Herstellung einer Tobramycin-Inhalationslösung mit einem Gehalt von 100 mg/ml

Als Ausgangsstoffe werden 11,08 g Tobramycin, 5,41 g Schwefelsäure (96 %), 0,2 g Natriumchlorid und 90,95 g Wasser für Injektionszwecke eingesetzt. Alle Schritte finden unter aseptischen Bedingungen und unter Stickstoffbegasung statt. Das Wasser wird vorgelegt, in das zunächst die Schwefelsäure gegeben wird. Anschließend werden nacheinander das Natriumchlorid und der Wirkstoff zugegeben. Der Ansatz wird bis zur visuell kontrollierten, vollständigen Auflösung aller festen Komponenten gerührt. Dabei werden ca. 100 ml einer Lösung erhalten, die einen pH-Wert von etwa 6,0, eine Osmolalität von etwa 0,22 Osmol/l, eine dynamische Viskosität von etwa 1,9 mPa·s und eine Oberflächenspannung von etwa 71 mN/m aufweist. Die Lösung wird sterilfiltriert und in eine Infusionsflasche mit einem Füllvolumen von 100 ml abgefüllt. Die Flasche wird mit einem durchstechbaren Elastomerstopfen dicht verschlossen und mit einer Alukappe gesichert.

### Beispiel 2: Verneblung einer Tobramycin-Inhalationslösung mit einem Gehalt von 100 ma/ml mit einem piezoelektrischen Vernebler

Von der nach Beispiel 1 hergestellte Lösung werden mit einer sterilen Kanüle und Einmalspritze 2 ml aseptisch entnommen und in das Vorratsgefäß eines piezoelektrischen Verneblers vom Typ eFlow^{™} (Fa. PARI) gegeben. Zur Erzeugung des Aerosols wurde das Gerät nach Bedienungsanleitung betrieben. Das Aerosol wurde mit Laser Diffraction (Malvern MasterSizer X) und in einem Andersen Kaskadenimpaktor auf Lungengängigkeit geprüft. Zur Verneblung wurden 3,2 Minuten benötigt. Die per Laser Diffraction gemessene Fraktion der Partikel bis 5 µm betrug 75 %, die mit dem Kaskadenimpaktor bestimmte Fraktion bis 5 µm betrug 77 %.

### Beispiel 3: Herstellung einer tensidhaltigen Tobramycin-Inhalationslösung

Als Ausgangsstoffe werden 10,88 g Tobramycin, 5,41 g Schwefelsäure (96 %), 0,2 g Natriumchlorid, 0,1 g Tween^{®} 80 und 90,95 g Wasser für Injektionszwecke eingesetzt. Alle Schritte finden unter aseptischen Bedingungen und unter Stickstoffbegasung statt. Das Wasser wird vorgelegt, in das zunächst die Schwefelsäure gegeben wird. Anschließend wird bei Raumtemperatur Tobramycin zugegeben und gelöst. In diese Lösung werden Natriumchlorid und Tween^{®} gegeben. Der Ansatz wird gerührt, bis eine klare Lösung entsteht. Es werden ca. 100 ml einer Lösung erhalten, die einen pH-Wert von etwa 6,2, eine Osmolalität von etwa 0,22 Osmol/l, eine dynamische Viskosität von etwa 1,9 mPa·s und eine Oberflächenspannung von etwa 43 mN/m aufweist. Die Lösung wird sterilfiltriert und aseptisch zu je 2 ml in Einzeldosisbehältnisse aus Polypropylen gefüllt.

### Beispiel 4: Herstellung einer Tobramycin-Inhalationslösung mit 2 Tensiden

Als Ausgangsstoffe werden 10,88 g Tobramycin, 5,41 g Schwefelsäure (96 %), 0,2 g Natriumchlorid, 0,45 g DMPC, 0,91 g Tyloxapol und 89,59 g Wasser für Injektionszwecke eingesetzt. Zunächst werden DMPC und Tyloxapol in dem Wasser dispergiert. Dieser Ansatz wird danach bei 1500 bar hochdruckhomogenisiert, bis eine opaleszente Lösung entsteht. Anschließend werden die Schwefelsäure und der Wirkstoff zugegeben, wodurch es zunächst zur Ausfällung kommt, die aber nach 24 h Rühren bei Raumtemperatur nicht mehr zu beobachten ist, sondern allenfalls ein Opaleszenz. Schließlich wird Natriumchlorid zugegeben, die Lösung sterilfiltriert und in Einzeldosisbehältnisse abgefüllt. Die Lösung hat einen pH von etwa 6,2, eine Oberflächenspannung von etwa 36,5 mN/m, eine dynamische Viskosität von etwa 2,07 mPa·s und eine Osmolalität von etwa 0,23 Osmol/l.

### Beispiel 5: Herstellung einer Tobramycin-Inhalationslösung mit CaCl₂-Zusatz

Als Ausgangsstoffe werden 10,88 g Tobramycin, 5,41 g Schwefelsäure (96 %), 0,2 g Natriumchlorid, 0,07 g Calciumchlorid und 90,95 g Wasser für Injektionszwecke eingesetzt. Das Wasser wird vorgelegt, in das zunächst die Schwefelsäure gegeben wird. Anschließend werden nacheinander der Wirkstoff und dann das Natriumchlorid gemeinsam mit dem Calciumchlorid zugegeben. Der Ansatz wird bis zur visuell kontrollierten, vollständigen Auflösung aller festen Komponenten gerührt. Dabei kann es nach der Zugabe der Salze zu einer vorübergehenden Ausfällung kommen, die allerdings nach 12stündigem Rühren nicht mehr zu beobachten ist. Die Lösung wird sterilfiltriert und in Einzeldosisbehältnisse abgefüllt. Die Zubereitung besitzt einen pH von etwa 6,0, eine Oberflächenspannung von etwa 70,2 mN/m, eine Viskosität von etwa 1,87 mPa·s und eine Osmolalität von etwa 0,24 Osmol/kg.

### Beispiel 6: Herstellung einer Tobramycin-Inhalationslösung mit MgSO₄-Zusatz

Als Ausgangsstoffe werden 10,88 g Tobramycin, 5,41 g Schwefelsäure (96 %), 0,2 g Natriumchlorid, 0,12 g Magnesiumsulfatheptahydrat und 90,95 g Wasser für Injektionszwecke eingesetzt. Die Herstellung der Lösung geschieht analog Beispiel 5. Die Lösung wird sterilfiltriert und in Einzeldosisbehältnisse abgefüllt. Die Zubereitung besitzt einen pH von etwa 6,1, eine Oberflächenspannung von etwa 69,8 mN/m, eine Viskosität von etwa 1,86 mPa·s und eine Osmolalität von etwa 0,24 Osmol/kg.

### Beispiel 7: Verneblung einer Tobramycin-Inhalationslösung mit einem Schwingmembranvernebler und Charakterisierung des Aerosols im Kaskadenimpaktor und Atemzusimulator.

Von einer analog Beispiel 1 hergestellten erfindungsgemäßen Tobramycinlösung wurden 1,4 ml entnommen und mit einem piezoelektrischen Vernebler (Schwingmembranvernebler) vom Typ eFlow^{™} (Fa. PARI GmbH) vernebelt und das Aerosol in einem Anderson Kaskadenimpaktor (ACI) und einem Atemzugsimulator vom Typ PARI COMPAS^{™} (15 Atemzüge/min, 500 ml A-temvolumen, Verhältnis Einatmung : Ausatmung 1 : 1) charakterisiert und mit der Verneblung einer handelsüblichen Tobramycinlösung (TOBI^{™} 300, 5 ml) in einem Düsenvernebler vom Typ PARI LC PLUS^{®} verglichen. Darüber hinaus wurde die geometrische Tröpfchengrößenverteilung der Aerosole durch Photonenkorrelationsspektroskopie (PCS) mit einem Malvern Master-SizerX bestimmt. Die Ergebnisse sind in Tabelle 1 zusammengefasst.

**Tabelle 1**

| Inhalationslösung | Nach Beispiel 6 (1,4 ml) | | TOBI 300 mg (5,0 ml) | |
|---|---|---|---|---|
| Vernebler | PARI eFlow^{™} | | PARI LC PLUS^{®} | |
| | Mittelwert | SD (n=3) | Mittelwert | SD (n=3) |
| MMD (PCS) [µm] bei 20 l/min | 3,89 | 0,10 | 3,90 | 0,05 |
| GSD [µm] | 1,50 | 0,01 | 2,02 | 0,02 |
| FPF [%<5 µm] | 73,8 | 2,1 | 64,4 | 0,6 |
| TOR [mg/min] | 521,2 | 45,7 | 465,3 | 13,3 |
| MMAD (ACI) [µm] bei 28,3 l/min | 3,89 | 0,14 | 3,39 | 0,12 |
| GSD [µm] | 1,48 | 0,04 | 2,15 | 0,01 |
| FPF [%<5 µm] | 71,5 | 0,5 | 71,1 | 0,1 |
| DD [mg] | 95,3 | 1,78 | 112,9 | 4,10 |
| Wirkstoffverlust im Vernebler [mg] | 12,0 | 0,90 | 133,2 | 7,87 |
| Wirkstoffverlust bei Verneblung [mg] | 26,4 | 2,91 | 52,3 | 4,52 |
| Verneblungsdauer [min] | 3,0 | 0,22 | 15,3 | 1,50 |
| DD [% der Dosis] | 68,9 | 1,26 | 37,2 | 1,36 |
| Wirkstoffverlust im Vernebler [% der Dosis] | 8,7 | 0,65 | 43,9 | 2,59 |
| Wirkstoffverlust bei Verneblung [% der Dosis] | 19,0 | 2,10 | 17,3 | 1,49 |
| Bilanz [% der Dosis] | 96,6 | 0,68 | 98,4 | 0,67 |
| RD [% der DD<5 µm] | 70,3 | 2,52 | 72,7 | 3,10 |
| RD [% der Dosis<5 µm] | 50,8 | 1,82 | 24,0 | 1,03 |
| DDR [mg/min] | 31,4 | 2,27 | 7,4 | 0,78 |
| DDR [% der Dosis/min] | 22,7 | 1,65 | 2,5 | 0,26 |
| RDDR [mg<5 µm/min] | 23,1 | 1,13 | 4,8 | 0,50 |

| | | | | |
|---|---|---|---|---|
| Erläuterungen: MMD: Gewichtsmittlerer Durchmesser (mass median diameter) GSD: Geometrische Standardabweichung (geometric standard deviation) FPF: Teilchenfraktion < 5 µm (fine particle fraction) TOR: Aerosolausbringungsrate (total output rate) MMAD: Gewichtsmittlerer aerodynamischer DD: Ausgebrachte Dosis (delivered dose) RD: Inhalierbare Dosis (respirable dose) DDR: Ausbringungsrate des Wirkstoffs (drug delivery rate) RDDR: Ausbringungsrate der inhalierbaren Wirkstoffs (respirable drug delivery rate) | | | | |

## Patentansprüche

1. Sterile, flüssige Zubereitung in Form einer wässrigen Lösung für die Applikation als Injektionslösung oder Aerosol enthaltend 80 mg/ml bis 120 mg/ml Tobramycin und einen sauren Hilfsstoff, **dadurch gekennzeichnet, dass** sie maximal 2 mg/ml Natriumchlorid enthält.

2. Zubereitung nach Anspruch 1, **dadurch gekennzeichnet, dass** sie weitgehend frei von Natriumchlorid ist.

3. Zubereitung nach Anspruch 2, **dadurch gekennzeichnet, dass** sie mindestens ein weitgehend neutrales Isotonisierungsmittel enthält.

4. Zubereitung nach Anspruch 3, **dadurch gekennzeichnet, dass** das lsotonisierungsmittel ein Magnesiumsalz, ein Calciumsalz, ein Zucker oder ein Zuckeralkohol ist.

5. Zubereitung nach einem der voranstehenden Ansprüche, **gekennzeichnet durch** einen pH-Wert von 5,5 bis 6,5.

6. Zubereitung nach einem der voranstehenden Ansprüche, **dadurch gekennzeichnet, dass** der saure Hilfsstoff Schwefelsäure oder Salzsäure ist.

7. Zubereitung nach einem der voranstehenden Ansprüche, **dadurch gekennzeichnet, dass** sie mindestens einen oberflächenaktiven Hilfsstoff enthält.

8. Zubereitung nach Anspruch 7, **dadurch gekennzeichnet, dass** der oberflächenaktive Hilfsstoff ein Phospholipid ist.

9. Zubereitung nach Anspruch 8, **dadurch gekennzeichnet, dass** sie als weiteren oberflächenaktiven Hilfsstoff Tyloxapol enthält.

10. Zubereitung nach einem der voranstehenden Ansprüche, **dadurch gekennzeichnet, dass** sie bei Raumtemperatur eine dynamische Viskosität von 1,6 bis 2,0 mPa·s und eine Osmolalität von 200 bis 300 mosmol/l besitzt.

11. Zubereitung nach einem der voranstehenden Ansprüche, **dadurch gekennzeichnet, dass** ihre Osmolalität 230 bis 280 mOsmol/l beträgt.

12. Zubereitung nach einem der voranstehenden Ansprüche, **dadurch gekennzeichnet, dass** sie in Form einer bemessenen Einzeldosis innerhalb einer Primärverpackung vorliegt.

13. Zubereitung nach Anspruch 12, **dadurch gekennzeichnet, dass** die Primärverpackung durch ein Kunststoffbehältnis gebildet wird, welches ein entfernbares Verschlusselement umfasst.

14. Zubereitung nach Anspruch 13, **dadurch gekennzeichnet, dass** im Kunststoffbehältnis durch die Entfernung des Verschlusselements eine runde Öffnung entsteht, deren Durchmesser etwa dem Innendurchmesser eines weiblichen Luer-Anschlusses entspricht.

15. Zubereitung nach Anspruch 13 oder 14, **dadurch gekennzeichnet, dass** das Kunststoffbehältnis nach Entfernung des Verschlusselements mit einem für die Zufuhr von Flüssigkeit vorgesehenen Anschlussstück eines Verneblers weitgehend schlüssig verbindbar ist.

16. Zubereitung nach einem der Ansprüche 13 bis 15, **dadurch gekennzeichnet, dass** das Kunststoffbehältnis mit mindestens einer Prägung versehen ist, welche eine Produktbezeichnung, einen Chargencode, ein Haltbarkeitsdatum und/oder eine Volumen- oder Dosismarke darstellt.

17. Kit zur Herstellung einer Zubereitung nach einem der voranstehenden Ansprüche, enthaltend (a) eine flüssige oder feste wirkstoffhaltige und (b) eine flüssige, wirkstofffreie Komponente.

18. Verwendung einer Zubereitung nach einem der Ansprüche 1 bis 16 oder eines Kits nach Anspruch 17 zur Herstellung eines Arzneimittels zur intravenösen, intraarteriellen, subkutanen oder intramuskulären Injektion.

19. Verwendung einer Zubereitung nach einem der Ansprüche 1 bis 16 oder eines Kits nach Anspruch 17 zur Herstellung eines Arzneimittels zur Applikation in Form eines Aerosols.

20. Verwendung nach Anspruch 19 zur pulmonalen Applikation mit Hilfe eines Düsen-, Ultraschall- oder piezoelektrischen Verneblers.

21. Verwendung nach Anspruch 20, wobei der piezoelektrische Vernebler ein Gerät vom Typ eFlow^{™} der Firma PARI ist.

22. Verwendung nach Anspruch 21 zur nasalen Applikation mit Hilfe eines mechanischen Zerstäubers oder eines Düsen-, Ultraschall- oder piezoelektrischen Verneblers.

23. Verwendung nach Anspruch 22 zur Applikation an die Schleimhäute der Nasenneben- und/oder Stirnhöhlen.

24. Verwendung nach Anspruch 22 zur Applikation mit Hilfe eines Düsenverneblers, der ein Nasenstück für die Zuführung von Aerosol in einen oder beide Nasenflügel eines Patienten enthält und dessen Aerosolausströmung pulsierende Druckschwankungen aufweist.

## Claims

1. Sterile, liquid preparation in the form of an aqueous solution for the application as a solution for injection or as an aerosol containing 80 mg/ml to 120 mg/ml of tobramycin and an acidic adjuvant, **characterized in that** the preparation contains not more than 2 mg/ml of sodium chloride.

2. Preparation according to claim 1, wherein the preparation is substantially free of sodium chloride.

3. Preparation according to claim 2, wherein the preparation contains at least one substantially neutral isotonising agent.

4. Preparation according to claim 3, wherein the isotonising agent is a magnesium salt, a calcium salt, a sugar or a sugar alcohol.

5. Preparation according to one of the preceding claims, wherein the preparation has a pH of 5.5 to 6.5.

6. Preparation according to one of the preceding claims, wherein the acidic adjuvant is sulfuric acid or hydrochloric acid.

7. Preparation according to one of the preceding claims, wherein the preparation contains at least one surface active adjuvant.

8. Preparation according to claim 7, wherein the surface active adjuvant is a phospholipid.

9. Preparation according to claim 8, wherein the preparation contains tyloxapol as a further surface active adjuvant.

10. Preparation according to one of the preceding claims, wherein the preparation has a dynamic viscosity at room temperature of 1.6 to 2.0 mPa·s and an osmolality of 200 to 300 mOsmol/l.

11. Preparation according to one of the preceding claims, wherein the preparation has an osmolality of 230 to 280 mOsmol/l.

12. Preparation according to one of the preceding claims, wherein the preparation exists as a measured single dose within a primary packaging.

13. Preparation according to claim 12, wherein the primary packaging is formed by a plastic container which comprises a removable closure element.

14. Preparation according to claim 13, wherein the removal of the closure element forms a round opening in the plastic container, the diameter of which corresponds to about the internal diameter of a female Luer lock adapter.

15. Preparation according to claim 13 or 14, wherein the plastic container, after removal of the closure element, can be fitted essentially tightly to the connector of a nebuliser which is provided for the input of liquid.

16. Preparation according to one of claims 13 to 15, wherein the plastic container is provided with at least one embossing, which represents a product designation, a lot code, a use-by date and/or a volume or dose marking.

17. Kit for the manufacture of a preparation according to one of the preceding claims, comprising (a) a liquid or solid component containing an active agent and (b) a liquid component which is free of active agent.

18. Use of a preparation according to one of claims 1 to 16 or of a kit according to claim 17 for the manufacture of a medicament for intravenous, intraarterial, subcutaneous or intramuscular injection.

19. Use of a preparation according to one of claims 1 to 16 or of a kit according to claim 17 for the manufacture of a medicament for the application in the form of an aerosol.

20. Use according to claim 19 or the pulmonary application by means of a jet, ultrasonic or piezoelectric nebuliser.

21. Use according to claim 20, wherein the piezoelectric nebuliser is a device of the eFlow^{™} type of PARI.

22. Use according to claim 21 for the nasal application by means of a mechanical atomizer or a jet, ultrasonic or piezoelectric nebuliser.

23. Use according to claim 22 for administration to the mucosa of the paranasal and/or frontal sinuses.

24. Use according to claim 22 for administration by means of a jet nebuliser which comprises a nose piece for supplying an aerosol to one or both nostrils of a patient and the aerosol output of which has a pulsating pressure.

## Revendications

1. Composition liquide stérile, sous la forme d'une solution aqueuse pour l'application en tant que solution à injecter ou aérosol, contenant 80 mg/ml à 120 mg/ml de tobramycine et un auxiliaire acide, **caractérisée en ce qu'**elle contient au maximum 2 mg/ml de chlorure de sodium.

2. Composition selon la revendication 1, **caractérisée en ce qu'**elle est largement exempte de chlorure de sodium.

3. Composition selon la revendication 2, **caractérisée en ce qu'**elle contient au moins un agent isotonisant largement neutre.

4. Composition selon la revendication 3, **caractérisée en ce que** l'agent isotonisant est un sel de magnésium, un sel de calcium, un sucre ou un alcool de sucre.

5. Composition selon l'une quelconque des revendications précédentes, **caractérisée par** une valeur de pH de 5,5 à 6,5.

6. Composition selon l'une quelconque des revendications précédentes, **caractérisée en ce que** l'auxiliaire acide est l'acide sulfurique ou l'acide chlorhydrique.

7. Composition selon l'une quelconque des revendications précédentes, **caractérisée en ce qu'**elle contient au moins un auxiliaire tensioactif.

8. Composition selon la revendication 7, **caractérisée en ce que** l'auxiliaire tensioactif est un phospholipide.

9. Composition selon la revendication 8, **caractérisée en ce qu'**elle contient comme autre auxiliaire tensioactif du Tyloxapol.

10. Composition selon l'une quelconque des revendications précédentes, **caractérisée en ce qu'**elle possède à température ambiante une viscosité dynamique de 1,6 à 2,0 mPa.s et une osmolalité de 200 à 300 mOsmol/l.

11. Composition selon l'une quelconque des revendications précédentes, **caractérisée en ce que** son osmolalité atteint 230 à 280 mOsmol/l.

12. Composition selon l'une quelconque des revendications précédentes, **caractérisée en ce qu'**elle se présente sous la forme d'une dose unique mesurée à l'intérieur d'un conditionnement primaire.

13. Composition selon la revendication 12, **caractérisée en ce que** le conditionnement primaire est formé par un récipient en plastique, qui comprend un élément de fermeture amovible.

14. Composition selon la revendication 13, **caractérisée en ce que**, dans le récipient en plastique, l'enlèvement de l'élément de fermeture fait naître une ouverture ronde dont le diamètre correspond environ au diamètre interne d'un raccord luer femelle.

15. Composition selon la revendication 13 ou 14, **caractérisée en ce que** le récipient en plastique peut être raccordé largement par engagement avec une pièce de raccord d'un atomiseur prévue pour l'apport de liquide après retrait de l'élément de fermeture.

16. Composition selon l'une quelconque des revendications 13 à 15, **caractérisée en ce que** le récipient en plastique est doté d'au moins une empreinte, qui représente une désignation de produit, un code de charges, une date de péremption et/ou une marque de volume ou de dose.

17. Kit pour la fabrication d'une composition selon l'une quelconque des revendications précédentes, contenant (a) un composant liquide ou solide contenant un principe actif et (b) un composant liquide, exempt de principe actif.

18. Utilisation d'une composition selon l'une quelconque des revendications 1 à 16 ou d'un kit d'après la revendication 17 pour la fabrication d'un médicament destiné à l'injection intraveineuse, intra-artérielle, sous-cutanée ou intramusculaire.

19. Utilisation d'une composition selon l'une quelconque des revendications 1 à 16 ou d'un kit selon la revendication 17 pour la fabrication d'un médicament pour l'application sous forme d'un aérosol.

20. Utilisation selon la revendication 19 pour l'application pulmonaire à l'aide d'un atomiseur à buses, à ultrasons ou piézoélectrique.

21. Utilisation selon la revendication 20, dans laquelle l'atomiseur piézoélectrique est un appareil du type eFlow^{™} de la société PARI.

22. Utilisation selon la revendication 21 pour l'application nasale à l'aide d'un pulvérisateur mécanique ou d'un atomiseur à buses, à ultrasons ou piézoélectrique.

23. Utilisation selon la revendication 22 pour l'application sur la peau des muqueuses des cavités nasales et/ou des sinus frontaux.

24. Utilisation selon la revendication 22 pour l'application à l'aide d'un atomiseur à buses, qui contient une pièce nasale pour la conduite de l'aérosol dans un ou les deux ailerons du nez d'un patient et dont la distribution de l'aérosol présente des oscillations de pression pulsantes.
